# EUROPEAN PATENT APPLICATION

(11) **EP 3 834 746 A1**
(43) Date of publication of application: **16.06.2021**
(21) Application number: 19861310.1
(22) Date of filing: 12.04.2019
(51) Int. Cl.: A61B 17/08

(54) **WOUND CLOSURE DEVICE**

(30) Priority: 20.09.2018 CN 201811101842
(71) Applicant: Shanghai Jinchen Medical & Pharmaceutical Technology Co., Ltd., Shanghai 200131 (CN)
(72) Inventor: ZHANG, Xinping, Nanchong, Sichuan 637000 (CN)
(74) Representative: Gulde & Partner
(86) International application number: PCT/CN2019/082548
(87) International publication number: WO 2020/057113

(57) **Abstract**

A wound closure apparatus pertaining to the field of skin wound sutures, comprising a window body, zippers, and fixators. The window body comprises a body wall and top wall. The zippers are set at both sides of the window body. One end of the zippers are connected to the fixators, and the other end are connected to the window body. fixed adhesive layers are set on the bottom surface of the fixators facing the skin. The wound closure apparatus enables functional modules necessary for wound treatment to reliably, continuously, and directly act on the treatment area, effectively preventing exudate accumulation from causing the suture device to fall off the wound, secondary infection, suture failure, or staggered malunion, allowing for better physiological regeneration and healing.

## Description

### Field of the Invention

The invention relates to a wound closure apparatus, in particular to a wound closure apparatus in the field of skin wound suture.

### Background of the Invention

In the fields of human anatomy and tissue repair, skin tissue is the most basic and important barrier and enclosure structure between the inside and outside of the human body, including epidermis, dermis and subcutis. When the barrier is damaged by surgical incision or trauma, surgical suture must be performed to restore free skin edges of wounds to the original layered apposition as soon as possible according to the original anatomical structure of the skin, and the barrier structure and physiological function of the original skin can be restored by regeneration of skin tissue. In order to promote layered apposition of free skin edges of wounds, precise apposition and levelling of skin edges of wounds in horizontal and vertical axial directions are particularly important for wound healing, which is the basis for physiological regeneration and healing of wounds. On this basis of the understanding, if various functional treatment modules for promoting healing can be effectively arranged at wound healing areas, such as wound exudate absorption module, wound hemostasis module, wound bacteriostatic and antibacterial module, sensor module for acquiring wound information and cell tissue regeneration module for promoting wound healing, physiological healing of wounds will be definitely accelerated and the physiological healing quality of wounds will be improved.

In the prior art, various types of apposition devices are used to solve the problem of apposition on both sides of wounds. However, such apposition devices can simply achieve the function of wound apposition and closure in the horizontal axial direction. Meanwhile, due to such apposition devices are directly arranged over wounds, medical staff are unable to freely and completely apply important functional treatment modules or units to wound healing areas located in the middle of wounds, such as wound exudate absorption module, wound skin edges apposition reference module, wound hemostasis module, wound bacteriostatic and antibacterial module, sensor module for acquiring wound information and cell tissue regeneration module for promoting wound healing. Therefore, the following defects are caused in practical clinical application of the existing apposition devices: I. lack of independent efficient module to clean bleeding and exudate from wounds in time, secondary wound infection due to accumulation of a large amount of exudate, suture device dislodgement, wound suture failure, delayed wound healing and non-healing wounds resulting from exudate accumulation; II. lack of consistently effective vertical apposition enforcement mechanism or apposition reference module, failure to ensure apposition and levelling of skin edges of wounds in the vertical axial direction, and failure to complete precise layered apposition of the skin, resulting in staggered malformed healing (e.g., step-like staggered healing, pimple-like healing and bad healing with scars); and III. lack of effective wound hemostasis module, wound bacteriostatic and antibacterial module, tissue regeneration promotion module or other sustained action in wound healing areas, or failure to ensure that the above modules reliably act on the wound healing areas consistently in practice. The actual efficiency is extremely low or actually missing. Therefore, it is difficult to achieve good physiological regeneration and healing of wounds.

### Summary of the Invention

The technical problem to be solved by the invention is to provide a more effective wound closure apparatus, in which a complete or interference-free treatment unit is constructed in a wound healing area located in the middle of a wound, so that functional modules required for wound treatment can act reliably, consistently and directly on the wound healing area, thereby effectively avoiding suture device dislodgement, secondary wound infection, wound suture failure and staggered malformed wound healing resulting from exudate accumulation, and achieving better physiological regeneration and healing of the wound.

The technical solution for solving the technical problem of the invention is as follows: a wound closure apparatus, comprising a window body, zippers and fixators, wherein the window body comprises a body wall and a top wall; zippers are arranged at both sides of the window body; one end of the zipper is connected with the fixator and the other end is connected with the window body, and fixing adhesive layers are formed on the bottom of fixators towards skin.

Furthermore, a window that can be opened and closed is arranged on the top wall of the window body.

Furthermore, an apposition reference support module is arranged in the window body, and the bottom of the apposition reference support module facing a wound is a rigid reference plane.

Furthermore, the wound closure apparatus further comprises a first area negative pressure sealing film and a second area negative pressure sealing film, wherein the first area negative pressure sealing film and the body wall enclose to form a window negative pressure sealing chamber; the second area sealing film, the outer wall of the window body and peripheral skin of the wound enclose to form a peripheral negative pressure sealing chamber; and fixators are positioned in the peripheral negative pressure sealing chamber.

Furthermore, the body wall is an annular closed isolation fence.

Furthermore, a shaper is attached to the isolation fence, the geometric shape of the shaper determines the final geometric shape of the isolation fence, and is prefabricated into a closed ring shape extending a certain distance to both sides along the actual longitudinal axial direction of the wound.

Furthermore, the top wall of the window body is made of a flexible material and can sink downward under the action of a negative pressure. A window that can be opened and closed is arranged on the top wall, a sash that can be opened and closed is arranged on the window, the top wall of the window body is closed when the sash is closed, and the internal space of the window body is communicated with the external space when the sash is opened.

Furthermore, zippers comprise lock catches and spines, wherein the lock catch is connected to the fixator, a ratchet is arranged in the lock catch, one end of the spine is connected and fixed to the window body, and the other end is inserted into the lock catch. Furthermore, zippers can be configured as laces which are folded back at the connection with the window body to form folded parts, and the folded part of the lace is provided with an adhesive area to connect and fix to the surface of the fixator and the back of the lace.

Furthermore, fixators are arranged on both sides of the window body, and an adhesive layer is formed on the bottom of the fixator towards the skin for adhering and fixing to the surface of the peripheral skin on both sides of the wound. A base band parallel to the corresponding outer wall of the window body is arranged at an edges of the fixator toward the inside of the wound.

Furthermore, the functional modules comprise a wound exudate absorption module and/or a wound hemostasis module and/or a wound bacteriostatic and antibacterial module and/or a wound information acquisition module and/or a cell tissue regeneration module.

Furthermore, the wound closure apparatus further comprises a negative pressure pipeline that communicates the inner negative pressure sealing chamber and the peripheral negative pressure sealing chamber with the outside.

The advantageous effects of the invention are as follows: a window body structure corresponding to a wound skin edges healing area is creatively arranged in a functional wound healing area where a free skin edges in the middle of a wound is located, and an effective, reliable and stable solid structural isolation is achieved between the functional wound healing area and the peripheral non-functional wound healing area by arranging the window body structure, so as to effectively ensure that various important treatment modules in the window body structure can consistently act on the wound healing area more reliably, stably and directly, and function effectively without external adverse interference, and thus can promote good physiological healing of the wound. Meanwhile, a prefabricated shaper is arranged on the window body structure, which can further meet precise customization and effective isolation of functional healing areas for clinical wounds with various irregular geometric shapes, and further solve the suture problem of clinical wounds with irregular geometric shapes.

### Brief Description of the Drawings

Fig. 1 is a front view of the present invention.
Fig. 2 is a schematic diagram of the present invention for wounds with irregular geometric shapes.
Fig. 3 is a schematic diagram of zippers using ratchet structures of the present invention.
Fig. 4 is a schematic diagram of zippers using lace structures of the present invention.

The numerals in the drawings are represented as follows: a window body 1, zippers 2, fixators 3, a first area negative pressure sealing film 4, a second area negative pressure sealing film 5, a window negative pressure sealing chamber 6, a peripheral negative pressure sealing chamber 7, a apposition reference support module 8, a rigid reference plane 9, a shaper 10, base bands 11, lock catches 12, spines 13, laces 14, skin 15, a window 16.

### Detailed Description of the Preferred Embodiments

The present invention will be further described with reference to the accompanying drawings.

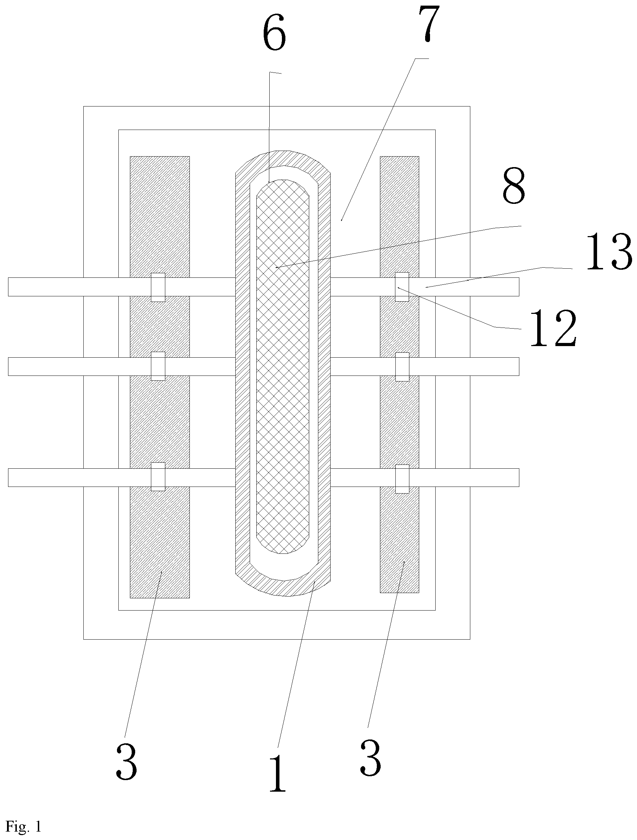

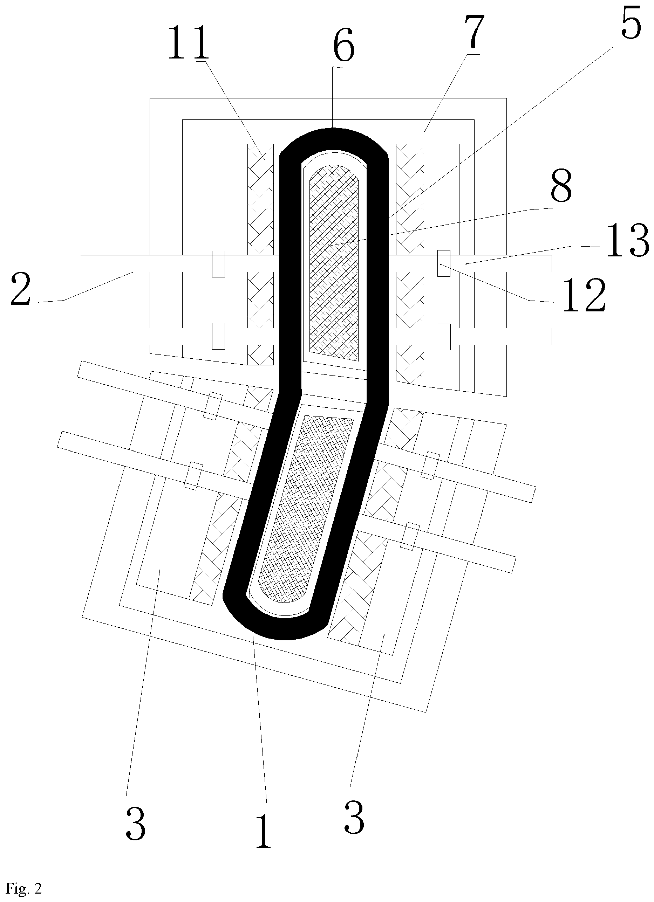

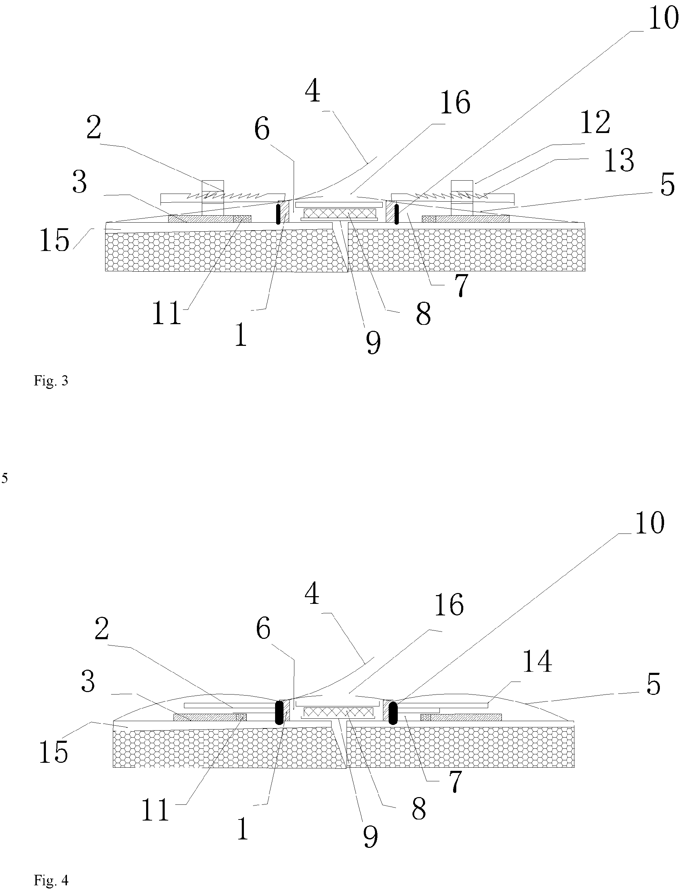

The wound closure apparatus as shown in Fig. 1, Fig. 2, Fig. 3 and Fig. 4 comprises a window body 1, zippers 2 and fixators 3, wherein the window body 1 comprises a body wall and a top wall; the zippers 2 are arranged at both sides of the window body 1; one end of the zippers 2 are connected with the fixators 3 and the other end are connected with the window body 1, and fixing adhesive layers are formed on one side of the fixators 3 towards skin 15.

Furthermore, a window 16 that can be opened and closed can be arranged on the top wall of the window body. In this way, corresponding operations can be performed easily in window body 1 by opening and closing the window 16 if necessary, for example, functional modules in the window body 1 can be incorporated or replaced through the window 16.

In the present invention, a window body 1 corresponding to a wound skin edges healing area is arranged in a functional wound healing area where a free skin edges in the middle of a wound is located, and an effective, reliable and stable solid structural isolation is achieved between the functional healing area of the wound skin edges and the peripheral non-functional wound healing area by arranging the window body 1 structure, so that various important functional modules in the window body 1 structure can consistently act on the wound healing area more reliably, stably and directly, and function more effectively without external adverse interference, thus can promote good physiological healing of the wound.

The fixators 3 are located on the skin 15 on both sides of the midline of the wound, and the skin 15 on both sides of the midline of the wound is fixed by adhesive layers. After the fixators 3 are connected with the zippers 2, the zippers 2 gets the fixators 3 and the skin 15 on both sides of the wound close to the window body 1, thereby driving skin edges on both sides of the wound to close toward the center of the wound.

The top wall of the window body is made of a flexible material and can sink downward under the action of a negative pressure. A window 16 that can be opened and closed is arranged on the top wall. When the window 16 is open, the interior of the window body 1 is open and visible, and functional modules in the window body 1 can be incorporated or replaced to realize visible open treatment of wound hemostasis, exudate and apposition of the wound edges on the skin 15 in the wound area.

When the window 16 on the top wall is open, an apposition reference support module 8 can be accurately placed on the wound surface, and the bottom of the apposition reference support module 8 facing the wound is a rigid reference plane 9, which can ensure that the top wall sinks downward under the synergistic action of the negative pressure of the window body 1 while the zippers 2 allow horizontal axial apposition of the wound skin edges toward the midline of the wound, so that the wound skin edges is closely attached to the rigid reference plane 9 of the apposition reference support module 8 in the vertical axial direction of the wound without dislocation and gap, forcing the wound skin edges to achieve layered apposition in the vertical axial direction of the wound, and achieve a standard flat state of the rigid reference plane 9, which can ensure layered apposition of the wound skin edges according to the original anatomical structure of the skin 15 in both the horizontal and vertical axial directions. The opening and closing structure of the window 16 can be realized by using a structure that can be opened and closed, such as a flip-open sash, a slide-open cover, a removable open cover, a snap-on cover or a zip. The shape of the window 16 can be designed into a rectangle, a circle, or a special shape adapted to the wound based on the length and shape of the wound or treatment modules to be placed. The window 16 can be flexibly set as integral opening and closing or sectional independent opening and closing depending if necessary.

The wound closure apparatus further comprises a first area negative pressure sealing film 4 and a second area negative pressure sealing film 5, wherein the first area negative pressure sealing film 4 and the body wall enclose to form a window negative pressure sealing chamber 6; the second area sealing film 5, the outer wall of the window body 1 and peripheral skin 15 enclose to form a peripheral negative pressure sealing chamber 7; and the fixators 3 are positioned in the peripheral negative pressure sealing chamber 7. In particular, two independent double negative pressure sealing chambers are formed from the first area negative pressure sealing film 4 and the second area negative pressure sealing film 5 in the present invention so as to facilitate independent operations in different areas and achieve different functional treatment purposes. In the independent window negative pressure sealing chamber 6, physicians can easily fix the functional modules in sequence or in combination in the wound healing area for wound treatment by simply opening and closing the window 16, so that the modules for wound treatment can exert their functions more stably, reliably, consistently and effectively without external adverse interference, and thus can promote good physiological healing of wounds. In the area of the peripheral negative pressure chamber 7, physicians can independently operate the laces 14 or the spines 13 of the zippers 2 to adjust the apposition of the peripheral skin 15. The apposition of the wound applied by a lock will not be affected due to the relief of the overall negative pressure when treatment operations related to the wound healing area are independently implemented. The wound apposition state maintained by the apposition reference support module 8 in the negative pressure chamber of the window body 1, and the therapeutic functions and effects consistently and stably exerted by the functional wound treatment modules in the window body 1 will not be affected when the locking force is adjusted independently. The wound closure apparatus with double negative pressure chambers can ensure that bleeding and exudate from the wound are completely removed in time, and that the apposition reference platform can be adhered closely to and pressed on the upper surface of the wound skin edges under the sustained action of negative pressure, thus avoiding suture device dislodgement, suture failure and secondary wound infection due to exudate accumulation.

In the present invention, the body wall is an annular closed isolation fence. The isolation fence can prevent the wound exudate from flowing out and infiltrating the fixators 3 and other structures outside the window body 1, causing suture device dislodgement, suture failure and secondary wound infection due to exudate accumulation, resulting in non-healing or delayed healing.

In the present invention, a shaper structure is especially attached to the isolation fence. The geometric shape of the shaper can be prefabricated according to the actual shape of a wound, and further flexibly prefabricated into a closed ring shape extending a certain distance to both sides of the wound along the actual midline of the wound to adapt to the actual shape of the wound. The geometric shape of the shaper determines the final geometric shape of the isolation fence, thus functional wound healing areas of various shapes can be accurately prefabricated and precise customization and effective isolation of the functional wound healing areas can be achieved, so as to ensure that the treatment modules arranged in the functional healing areas can consistently exert their functions more effectively, reliably, stably and directly without external adverse interference, and thus can promote good physiological healing of wounds. Meanwhile, by realizing the geometric shape of the prefabricated shaper, the wound closure apparatus can better adapt to the precise customization and effective isolation of functional healing areas for a large number of clinical non-linear wounds, such as "bending, S-shaped, L-shaped, Z-shaped" and other irregular wounds. The practical beneficial outcome is that the scope of application of the product for treating clinical wounds is greatly extended, and the problem of suturing irregular clinical wounds is further solved.

In the present invention, the zippers 2 are of ratchets structure or a laces 14 structure. The ratchets structure comprises lock catches 12 and spines 13, wherein the lock catches 12 are connected to the fixators 3, ratchets are arranged in the lock catches 12 to restrict the spines 13 from sliding outward in one direction, one end of the spines 13 are connected to the window body 1, and the other end are inserted into the lock catches 12. The ratchets of the lock catches 12 can restrict the spines 13 from sliding outward in one direction. It is also possible to pass the lock catches 12 and the spines 13 through the second area negative pressure sealing film 5, and the lock catches 12 and the spines 13 can be located outside the sealing film for the purpose of easy operation. The skin 15 on both sides of the wound can be contracted towards the middle by pulling the spines 13. The laces 14 structure comprises the laces 14 which are folded back at the connection with the window body 1 to form folded parts, and the folded parts of the laces 14 are provided with an adhesive area to connect and fix to the surface of the fixators 3 and the back of the laces 14. The laces 14 can be pulled reversely to drive the skin 15 to the middle, and the adhesive area of the laces 14 can be adhered and fixed to the back of the laces 14 and the adhesive structures on the upper surface of the fixators 3 at any position, so as to adjust the pull distance arbitrarily and make the operation easier and more convenient.

The laces 14 can be further configured in flat thin structures, and the flat laces 14 can be arranged in the peripheral negative pressure chamber more easily due to the limited space.

The fixators 3 are arranged on both sides of the window body 1, and adhesive layers are formed on the bottom of the fixators 3 toward the skin 15 for adhering and fixing to the surface of the peripheral skin 15 on both sides of the wound. base bands 11 parallel to the corresponding outer wall of the window body 1 are arranged at edges of the fixators 3 toward the inside of the wound.

In order to better suture a large number of non-linear irregular wounds that are difficult to be treated by conventional sutures clinically, such as "bending, S-shaped, L-shaped, Z-shaped" and other irregular wounds, the fixators 3 can be further arranged into separate sections, and the inner base band 11 of each separate section of the fixator 3 is placed parallel to the corresponding outer wall section of the window body 1.

The base bands 11 are rigid linear skeletons that can uniformly pull the skin 15 on both sides of the wound to close to the midline in parallel, and ensure even apposition of wound skin edges of each section when suturing an irregular wound. The base band 11 gathers and transmits the suturing force of each separate section to the corresponding section of the window body 1 through the zippers 2. Meanwhile, the base bands 11 also disperse the high suturing force gathered between the window body 1 and the fixators 3 linearly and uniformly to the fixators 3 and the surface of the skin 15 adhered thereto along the base bands 11, so as to avoid the high suturing stress of the closure apparatus from being too concentrated or focused on the local skin 15 of the wound which may result in the corresponding stress injury to the skin 15. For example, the suture tension during suturing is concentrated at the puncture points of the skin 15, which will inevitably cause cutting damage to the skin 15 or even suture runout and suture failure. If the wound is closed simply with a tension-reduced butterfly closure strip, severe tension vesicles and other skin 15 injuries will occur at roots of butterfly wings, resulting in discontinuation of treatment. Therefore, the arrangement of the base bands 11 can better avoid suture failure of the wound or discontinuation of treatment.

Functional treatment modules are arranged in the inner space of the window body 1, including a wound exudate absorption module that can absorb wound exudate in time, a wound hemostasis module that can help quickly stop bleeding from a wound, a wound bacteriostatic and antibacterial module that can effectively avoid bacterial growth, a wound information acquisition module that can acquire real-time wound information using a sensor, and a cell tissue regeneration module that can promote wound healing. The sash of the window body 1 is arranged for easy operation and to fix the treatment modules individually in sequence or in combination depending on actual conditions of wounds, or depending on practical clinical needs or actual functional needs at different stages of wound healing based on treatment needs, in order to give full play to the functions of the treatment modules, improve the wound treatment efficiency, and reasonably customize schemes for using different functional treatment modules in the wound healing process for symptomatic treatment and targeted treatment, so as to promote good physiological healing of wounds.

The wound closure apparatus further comprises a negative pressure pipeline that communicates the window negative pressure sealing chamber 6 and the peripheral negative pressure sealing chamber 7 with the outside. The negative pressure pipeline is provided with a valve and communicated with the outside of the negative pressure sealing film to ensure that bleeding and exudate from the wound can be removed in time, and that the apposition reference platform can be adhered closely to and pressed on the upper surface of the wound skin edges under the action of negative pressure. The wound closure apparatus with double negative pressure chambers can be adhered closely to the wound skin 15 without detachment.

In conclusion, a stable solid isolation window body 1 structure is arranged in the functional wound healing area, so that the built-in functional treatment modules can consistently exert the functional therapeutic effect more effectively, reliably, stably and directly. The arrangement of the apposition reference support module 8 further ensure the layered horizontal alignment and vertical apposition of the wound skin edges according to the original anatomical structure of the skin 15 in both the horizontal and vertical axial directions. The window 16 that can be opened and closed on the window body 1 further meets the needs of physicians to use required functional modules individually or in combination depending on actual conditions of wounds, and achieves personalized customization of wound healing schemes as well as symptomatic treatment and targeted treatment. The prefabricated shaper on the window body 1 can better meet the precise customization and effective isolation of functional healing areas for wounds with various irregular geometric shapes, and further solve the suture problem of clinical wounds with irregular geometric shapes. As a result, such adverse consequences or secondary damages as suture device dislodgement, wound suture failure and secondary wound infection resulting from exudate accumulation in other closure apparatuses can be effectively avoided, and good physiological regeneration and healing of wounds can be achieved with higher efficiency and better effect.

The present invention provides a more effective wound closure apparatus, in which a complete or interference-free treatment unit is constructed in a wound healing area located in the middle of a wound, so that functional modules required for wound treatment can act reliably, consistently and directly on the wound healing area. Specifically, a solid window body 1 structure is used to isolate the functional healing area in the middle of the wound from the peripheral adhesive fixation area of the wound, so that various important functional modules capable of promoting good physiological healing of the wound are independently arranged in the window body 1 structure, and the functional modules in the window body 1 are isolated without interference from the peripheral area, and can act consistently on the wound healing area reliably, stably and directly, thereby effectively avoiding suture device dislodgement, secondary wound infection, wound suture failure and staggered malformed wound healing resulting from exudate accumulation, and further achieving better physiological regeneration and healing of the wound.

## Claims

1. A wound closure apparatus, **characterized in that** it includes a window body (1), zippers(2), and fixators (3), wherein the window body (1) including a body wall and a top wall, the zippers (2) configured to be arranged at both sides of the window body (1), one side of the zippers (2) being connected to the fixators (3), and the other end being connected to the window body (1),the bottom surface of the fixators (3) facing the skin (15) having fixing adhesive layers.

2. The wound closure apparatus of claim 1 is **characterized in that** the top wall has a window (16) that can be opened and closed.

3. The wound closure apparatus of claim 1 is **characterized in that** the window body (1) has an apposition reference support module (8) within it, whose bottom surface, facing the wound, is a rigid reference plane (9).

4. The wound closure apparatus of claim 1 is **characterized in that** it also includes a first area negative pressure sealing film (4) and a second area negative pressure sealing film (5), wherein, the first area negative pressure sealing film (4) and the body wall enclose to form a window negative pressure sealing chamber 6, and the second area sealing film 5, the outer wall of the window body 1 and peripheral skin 15 enclose to form a peripheral negative pressure sealing chamber 7,the fixators (3) being located in the peripheral negative pressure sealing chamber (7).

5. The wound closure apparatus of claim 1 is **characterized in that** the window body outer wall is an annular closed isolation fence.

6. The wound closure apparatus of claim 5 is **characterized in that** a shaper (10) is attached to the isolation fence of the window body (1).

7. The wound closure apparatus of claim 2 is **characterized in that** the window (16) of the window body (1) has a sash that can be opened or closed.

8. The wound closure apparatus of claim 1 is **characterized in that** the zippers (2) are a ratchets or laces (14) structure, the ratchets structure including lock catches (12) and spines (13), the lock catches (12) being connected to the fixators (3), ratchets teeth being installed within the lock catches (12), and one end of the spines (13) being connected to the window body (1), and the other end are inserted into the lock catches (12), the laces structure including laces (14), wherein the laces (14) are folded at the point where it connects to the window body (1) to form folded parts, which are affixed to the fixators (3) and back of the laces (14) using adhesive.

9. The wound closure apparatus described in claim 1 is **characterized in that** base bands (11) are installed in the fixators (3) facing the skin edges inside the wound, parallel to the outer wall of the corresponding window body (1).

10. The wound closure apparatus described in claim 1 is **characterized in that** a functional module is installed within the window body (1), which may be a wound exudate absorption module and/or a hemostasis module and/or a wound bacteriostatic and antibacterial module and/or a wound information acquisition module and/or a cell tissue regeneration module.

11. The wound closure apparatus of claim 4 is **characterized in that** it also includes a negative pressure pipeline which connects the internal negative pressure sealing chamber and the peripheral negative pressure sealing chamber (7) with the outside.

12. The wound closure apparatus of claim 6 is **characterized in that** the shaper (10) is bendable.

13. The wound closure apparatus of claim 2 is **characterized in that** the window (16) is configured for sectional independent opening and closing.

14. The wound closure apparatus of claim 1 is **characterized in that** the fixators (3) are configured independently in sections.
